# EUROPÄISCHE PATENTANMELDUNG

(11) **EP 3 770 911 A1**
(43) Veröffentlichungstag der Anmeldung: **27.01.2021**
(21) Anmeldenummer: 19187760.4
(22) Anmeldetag: 23.07.2019
(51) Int. Cl.: G16H 20/30, A61B 5/103, G16H 40/63, A63B 24/00, G09B 19/00

(54) **VERFAHREN ZUR ECHTZEITÜBERWACHUNG EINES NUTZERS AN EINEM ÜBUNGSGERÄT MIT EINEM MOBILEN ENDGERÄT, ÜBERWACHUNGSSYSTEM ZUR ECHTZEITÜBERWACHUNG EINES NUTZERS WÄHREND EINES TRAININGS AN EINEM ÜBUNGSGERÄT, TRAININGSSYSTEM BESTEHEND AUS EINEM ÜBERWACHUNGSSYSTEM UND EINEM ÜBUNGSGERÄT SOWIE VERWENDUNG EINES ÜBERWACHUNGSSYSTEMS ODER EINES TRAININGSSYSTEMS ZUR DURCHFÜHRUNG DES ERFINDUNGSGEMÄSSEN VERFAHRENS**

(71) Anmelder: aktivKONZEPTE AG, 66386 St. Ingbert (DE)
(72) Erfinder: SCHÖNTHALER, Stefan, Dr., 66386 St. Ingbert (DE); LOHO, Christian, 66386 St. Ingbert (DE); NEU, Daniela, 66386 St. Ingbert (DE)
(74) Vertreter: dompatent von Kreisler Selting Werner - Partnerschaft von Patent- und Rechtsanwälten mbB

(57) **Zusammenfassung**

Die Erfindung betrifft ein Verfahren zur Echtzeitüberwachung eines Nutzers an einem Übungsgerät mit einem mobilen Endgerät, das die folgenden Verfahrensschritte umfasst:
a) Identifizieren des Übungsgerätes, an welchem ein Training erfolgen soll, mittels des mobilen Endgeräts des Nutzers oder Identifizieren des Nutzers über ein an dem Übungsgerät, an welchem ein Training erfolgen soll, angeordnetes mobiles Endgerät,
b) Erfassen von Sensordaten mit mindestens einer an dem Übungsgerät angeordneten Sensoreinrichtung während der Durchführung des Trainings,
c) Abgleichen der Sensordaten als Trainings-Ist-Werte mit in einer Datenbankeinheit abgelegten nutzerspezifischen Trainings-Soll-Werten für das Übungsgerät zur Generierung einer Echtzeitrückmeldung des Trainingsablaufes, und
d) Anleiten des Nutzers durch Ausgeben der Echtzeitrückmeldung zur Optimierung des Trainingsablaufes über das mobile Endgerät.

## Beschreibung

Die Erfindung betrifft ein Verfahren zur Echtzeitüberwachung eines Nutzers an einem Übungsgerät mit einem mobilen Endgerät, ein Überwachungssystem zur Echtzeitüberwachung eines Nutzers während eines Trainings an einem Übungsgerät, weiterhin ein Trainingssystem umfassend ein Überwachungssystem und ein Übungsgerät zur Durchführung eines Trainings sowie weiterhin die Verwendung eines Überwachungssystems oder eines Trainingssystems zur Durchführung eines Verfahrens zur Echtzeitüberwachung eines Nutzers an einem Übungsgerät mit einem mobilen Endgerät.

Aus dem Stand der Technik sind Übungsgeräte bekannt, an denen Nutzer trainieren können. Ebenfalls ist es bekannt, dass derartige Übungsgeräte mit Anzeigeeinrichtungen ausgestattet sind, welche aktuelle Leistungsparameter des Trainings wiedergeben.

Nachteilig an den aus dem Stand der Technik bekannten Geräte ist, dass zur ordnungsgemäßen Nutzung der Übungsgeräte vor der initialen Nutzung eine Einweisung der trainierenden Nutzer durch geschultes Personal, wie beispielsweise Sportlehrer erfolgen muss um ein effektives Training zu gewährleisten und Verletzungen aufgrund fehlerhaften Trainings zu vermeiden. Zusätzlich zu der vorgenannten initialen Einweisung ist auch bei erfahrenen Trainierenden eine regelmäßige Begleitung bzw. Kontrolle durch geschultes Personal sinnvoll um ein kontinuierliches Training unter Einhaltung der Trainingssollvorgaben sicherstellen, aber auch um das Training an die sich kontinuierlich verändernden Leistungsparameter des trainierenden Nutzers anzupassen. Nur ein optimal an die Leistungsfähigkeit des Nutzers angepasster Übungsplan und folglich angepasste Trainingssollwerte gewährleisten ein effektives und gesundes Training. Bei den bestehenden Übungsgeräten und Verfahren zur Überwachung der Nutzer von Übungsgeräten besteht der Nachteil, dass die Einweisungen an den Übungsgeräten und die Überwachung der Einhaltung der Trainingspläne manuell und unter hohen Personalaufwand erfolgt.

Ausgehend von dem vorgenannten Stand der Technik stellt sich die vorliegende Erfindung die Aufgabe ein Verfahren zur Echtzeitüberwachung eines Nutzers an einem Übungsgerät mit einem mobilen Endgerät sowie Überwachungssystem zur Echtzeitüberwachung eines Nutzers während eines Trainings an einem Übungsgerät, sowie ein Trainingssystem bereitzustellen, welche dem Nutzer eines Übungsgerätes die Durchführung eines gesunden und leistungsfördernden Trainings unter eigenständiger Einhaltung und Kontrolle von vorgegebenen Trainingssolldaten und -randbedingungen ermöglicht, ohne dass dieses eine kontinuierliche Begleitung durch geschultes Personal erfordert.

Erfindungsgemäß gelöst wird die Aufgabe durch ein erfindungsgemäßes Verfahren gemäß Anspruch 1, ein erfindungsgemäßes Überwachungssystem gemäß Anspruch 10, einem Trainingssystem gemäß Anspruch 14 und einer Verwendung gemäß Anspruch 15.

Das erfindungsgemäße Verfahren zur Echtzeitüberwachung eines Nutzers an einem Übungsgerät mit einem mobilen Endgerät, umfasst die Verfahrensschritte:
a) Identifizieren des Übungsgerätes, an welchem ein Training erfolgen soll, mittels des mobilen Endgeräts des Nutzers oder Identifizieren des Nutzers über ein an dem Übungsgerät, an welchem ein Training erfolgen soll, angeordnetes mobiles Endgerät,
b) Erfassen von Sensordaten mit mindestens einer an dem Übungsgerät angeordneten Sensoreinrichtung während der Durchführung des Trainings,
c) Abgleichen der Sensordaten als Trainings-Ist-Werte mit in einer Datenbankeinheit abgelegten nutzerspezifischen Trainings-Soll-Werten für das Übungsgerät zur Generierung einer Echtzeitrückmeldung des Trainingsablaufes, und
d) Anleiten des Nutzers durch Ausgeben der Echtzeitrückmeldung zur Optimierung des Traniningsablaufes über das mobile Endgerät.

Im Rahmen der Erfindung kann es sich bei dem Übungsgerät beispielsweise um ein Kraft-, Kardiogerät oder ähnliches handeln, wobei die vorgenannten Geräte im Fitness-, Heim- und/oder im medizinischen Bereich eingesetzt werden können.

Als mobiles Endgerät werden insbesondere eine Smartwatch, ein Smartphone, ein Tablet, ein Laptop oder ein ähnlich gearteter Gegenstand zur mobilen Datenverarbeitung verstanden.

Erfindungsgemäß wird unter dem Begriff des Trainings ein Prozess verstanden, bei welchem durch gezielte Maßnahmen auf den Organismus eingewirkt wird, derart, dass die individuelle Leistungsfähigkeit einer an einem Übungsgerät trainierenden Person gesteigert, erhalten, oder wiedergewonnen werden kann, wobei insbesondere ein altersbedingter Leistungsabfall hinausgeschoben oder verlangsamt werden kann.

Aufgrund der Echtzeitrückmeldung wird der an einem Übungsgerät trainierende Nutzer in die Lage versetzt unmittelbar bei der Ausführung des Trainings mögliche Fehler in der Durchführung des Trainings, insbesondere bei der Vornahme des Bewegungsablauf zu identifizieren und geeignete Maßnahmen zu treffen um das Training nach den gewünschten Sollvorgaben durchzuführen bzw. gegebenenfalls das Training unmittelbar abzubrechen. Hierdurch können Verletzungen des Nutzers aufgrund einer fehlerhaften Durchführung des Trainings sicher verhindert werden. Gleichzeitig wird durch die Anleitung des Nutzers sichergestellt, dass dieser ein effektives und auf die körperlichen Grundvoraussetzungen abgestimmtes Training vornimmt. Die Sollwerte werden nutzerspezifisch je Übungsgerät individuell festgelegt. So ist es beispielsweise denkbar, dass nach einem initialen Leistungstest durch einen Trainer ein Leistungsniveau und ein Trainingsplan für einen trainierenden Nutzer festgelegt und als Trainingssollwerte hinterlegt wird. Insbesondere können dabei auch über eine zeitliche Veränderung der Trainingssollwerte für die Nutzer individuelle Trainingspläne realisiert werden, welche automatisiert über das erfindungsgemäße Verfahren an den Nutzer über die Echtzeitrückmeldung weitergegeben werden und der Nutzer zu einer Sollwertkonformen Trainingsdurchführung angeleitet wird. Insbesondere können die während des Trainings ermittelten Sensordaten als Trainingsistwerte abgespeichert und analysiert werden, so dass in Abhängigkeit der Trainingsistwerte eines vorangegangenen Trainings automatisiert die Trainingssollwerte für eine darauffolgendes Trainings des Benutzers an dem Übungsgerät angepasst wird. Hierdurch wird es ermöglicht Leistungszuwächse und/oder Leistungsabfälle eines trainierenden Nutzers ohne manuellen Eingriff zu berücksichtigen und zur Anpassung des Trainings zu nutzen.

Im Rahmen des erfindungsgemäßen Verfahrens kann es vorgesehen sein, dass das Verfahren nach dem Verfahrensschritt a) und vor dem Verfahrensschritt b) zusätzlich die Verfahrensschritte umfasst, wonach auf der Datenbankeinheit abgelegte Trainingsrandbedingungen und vorzunehmende Einstellungen am Übungsgerät vor Trainingsbeginn über das mobile Endgerät für den Nutzer wiedergegeben werden, sowie Vornahme der vorzunehmenden Einstellungen durch Abgleichen der Sensordaten mit den auf der Datenbankeinheit abgelegten Trainingsrandbedingungen und vorzunehmende Einstellungen mittels einer Auswertungseinheit überwacht werden und das Training nach Abschluss der Vornahme der Einstellungen über das mobile Endgerät für den Nutzer des Übungsgerätes freigegeben wird. Als Trainingsrandbedingungen können dabei beispielsweise die Anzahl der durchzuführenden Wiederholungen an einem Übungsgerät, ein zu wählendes Gewicht für die Durchführung der Übungen oder ein an dem Übungsgerät zu absolvierender Bewegungsweg an einem Übungsgerät als auch beispielsweise die Haltungsposition an einem gewählten spezifischen Übungsgerät während der Durchführung der Übung angesehen werden.

Die Ausgabe und nachfolgende Überwachung vorzunehmender Einstellungen und Trainingsrandbedingungen vor Durchführung des eigentlichen Trainings erhöht weiterhin die Sicherheit während der Trainingsdurchführung durch den Benutzer und verhindert Verletzungen der trainierenden Personen. Insbesondere wird ein Training für den Benutzer erst freigegeben, wenn die vorgegebenen Einstellungen an dem Übungsgerät auch wirklich durch den Benutzer vorgenommen wurden. Hierdurch wird ohne Eingriff durch eine Aufsichtsperson ermöglicht, dass die an dem Übungsgerät trainierende Person noch vor Aufnahme des eigentlichen Trainings sicherstellt, dass das Trainingsgerät auf den spezifischen Nutzer ordnungsgemäß eingestellt wurde. Insbesondere wird eine Benutzung der Übungsgeräte von unberechtigten Dritten oder die Nutzung der Übungsgeräte außerhalb der vorgegebenen Trainingssollwerte verhindert.

Bei den Einstellungen am Übungsgerät können beispielsweise Personendetails des trainierenden Nutzers, wie insbesondere dessen Körpergröße und -gewicht, berücksichtigt werden. In Abhängigkeit der auf der Datenbank abgelegten Personendetails können die vorzunehmenden Einstellungen, wie beispielsweise Position einer Arm- oder Beinauflage, aus der Datenbankeinheit für das spezifische gewählte Übungsgerät abgerufen und über das mobile Endgerät für den Nutzer wiedergegeben werden.

Durch die Wiedergabe und Überwachung der Trainingsrandbedingungen und vorzunehmender Einstellungen wird sichergestellt, dass der trainierende Nutzer an dem Übungsgerät zunächst die richtige Haltungsposition als auch insbesondere die gewünschte Trainingsintensität, wie beispielsweise Gewichtsbelastung, nicht überschreitet. Hierdurch wird ebenfalls sichergestellt, dass eine Überforderung und eine damit möglicherweise einhergehende Verletzung des trainierenden Nutzers verhindert wird.

Erfindungsgemäß kann es vorgesehen sein, dass im Verfahrensschritt c) vor dem Abgleichen der Sensordaten durch die Datenbankeinheit die Sensordaten mittels einer Auswertungseinheit interpretiert, gefiltert und sortiert werden und in einem nutzerspezifischen Datensatz der Datenbankeinheit abgelegt und darauffolgend der Datensatz mit den nutzerspezifischen Trainings-Soll-Werten abgeglichen wird zur Generierung der Echtzeitrückmeldung.

Hierbei können beispielsweise im Rahmen der Filterung der Sensordaten aus der Vielzahl der Sensordaten sogenannte Datenausreißer, welche signifikant von den umgebenden Daten abweichen oder von dem sonstigen Verlauf der Daten abweichen, aussortiert werden. Weiterhin können die Sensordaten über einen Zeitabschnitt gesammelt zur Erzielung einer Datenreduktion beispielweise das arithmetische Mittel der Datenpunkte oder deren Median ermittelt werden. Im Rahmen einer Sortierung können die ermittelten Sensordaten beispielsweise nach deren Werten oder Absolutwerten geordnet werden.

In einer bevorzugten Ausführungsform des Verfahrens können als Sensordaten die Position und/oder die zurückgelegten Wege eines definierten Objektes des Übungsgeräts und/oder die Geschwindigkeit, Winkellage oder Beschleunigung eines definierten Objektes des Übungsgeräts über die Zeitdauer des Trainingsablaufs mittels der mindestens einen Sensoreinrichtung erfasst werden.

Im Rahmen der vorliegenden Erfindung wird als definiertes Objekt ein Gegenstand des Übungsgerätes verstanden, welcher sich während des Trainings an dem Übungsgerät beispielsweise gegenüber der ortsunveränderlichen Grundstruktur des Übungsgerätes bewegt und anhand dessen der eigentliche Bewegungsablauf während des Trainings nachvollziehbar sowie über die mindestens eine Sensoreinrichtung messbar ist. Beispielsweise kann es sich bei dem definierten Objekt um den Absteckpin für die Gewichtseinstellung eines Übungsgerätes handeln, wobei der Absteckpin während der Durchführung des Trainings an dem Übungsgerät zusammen mit den eingestellten Gewichten verlagert wird.

Die Erfassung des Bewegungsablaufes bzw. der weiteren Parameter des durchgeführten Trainings über ein definiertes Objekt ermöglicht es das erfindungsgemäße Verfahren an jeglichen handelsüblichen Übungsgeräten zu realisieren. Insbesondere kann die Sensoreinrichtung nach initialer Anbringung an dem jeweiligen Übungsgerät einmalig kalibriert werden um beispielsweise den maximal möglichen Bewegungsweg zu detektieren und mögliche maximale Wendepunkte eines Bewegungsablaufes festzulegen.

In einer besonders bevorzugten Ausführungsform kann es vorgesehen sein, dass die Ausgabe der Echtzeitrückmeldung des Trainingsablaufes, der Trainingsrandbedingungen und/oder vorzunehmender Einstellungen optisch, haptisch und/oder akustisch über das mobile Endgerät erfolgt. Eine haptische oder akustische Ausgabe der Echtzeitrückmeldung ist vorteilhaft, da der trainierende Nutzer den Blick von dem mobilen Endgerät abwenden kann und trotzdem über mögliche Abweichungen des Trainingsablaufes in Echtzeit informiert ist und damit auch ohne Blickkontakt die ordnungsgemäße Vornahme des Trainings an dem Übungsgerät stets gewährleistet wird. Insbesondere können bei dem Auftreten von Abweichungen der Trainingsistwerte von den Trainingssollwerten bei der Überschreitung zuvor definierter Schwellwerte akustische Signale ausgegeben werden, welche den Nutzer dazu veranlassen den Blickkontakt mit dem mobilen Endgerät aufzunehmen um dort optische Anweisungen im Hinblick auf die Rückführung des Trainings innerhalb der Grenzen der Trainingssollwerte zu erreichen. Weiterhin können bei einer ordnungsgemäßen Durchführung des Trainings unter Einhaltung der Trainingssollwerte motivierende akustische Ausgaben über das mobile Endgerät erfolgen, insbesondere können zuvor abgespeicherte Sprachaufzeichnungen dem Benutzer wiedergegeben werden.

Bei der mindestens einen Sensoreinrichtung kann es sich um einen Kraftsensor, Entfernungssensor, Geschwindigkeitssensor, Rotationssensor, Winkelsensor und/oder einen Beschleunigungssensor handeln.

Bei dem Entfernungssensor bevorzugt um einen Laserentfernungssensor, einen Seilzugsensor oder ähnliches handeln.

Es kann weiterhin vorgesehen sein, dass die Echtzeitüberwachung bei der Durchführung eines Trainings mit mindestens einem kardiovaskulären Trainingsanteil erfolgt, wobei im Verfahrensschritt b) als zusätzliche Sensordaten mindestens ein Leistungsparameter am Übungsgerät erfasst wird, wobei der mindestens eine Leistungsparameter gewählt ist aus der Gruppe von Umdrehungszahl, Wattzahl, Steigung, Geschwindigkeit, Beschleunigung, Gang/Stufe, Puls, errechneter Kalorienverbrauch (kcal), Trainingsdauer, aufgenommene Sauerstoffmenge in Litern pro Minute (VO2), aufgenommene maximale Sauerstoffmenge (V02max), Seitigkeit links/rechts und/oder Wiederholungen und wobei der mindestens eine Leistungsparameter als ergänzende Information im Verfahrensschritt d) bevorzugt ohne vorherige Durchführung des Verfahrensschrittes c) dem Nutzer über das mobile Endgerät zusätzlich als Echtzeitrückmeldung ausgegeben wird. Als Leistungsparameter im kardiovaskulären Trainingsanteil kann zusätzlich manuell der Laktatwert zu definierten Zeitpunkten gemessen und dem Nutzer über das mobile Endgerät wiedergegeben werden.

Innerhalb des kardiovaskulären Trainingsanteils können Belastungspausen definiert und den Trainingsphasen einzelne Leistungsparameter zugeordnet werden.

Weiterhin ist es möglich, innerhalb des kardiovaskulären Trainingsanteils Leistungstests wie beispielsweise einen physical working capacity (pwc)-Test durchzuführen.

Erfindungsgemäß kann es vorgesehen sein, dass das Identifizieren des Übungsgeräts oder des Nutzers über die manuelle Eingabe eines Identifikationscodes an dem mobilen Gerät oder über das Scannen eines QR-Codes, NFC-Tags oder eines Transponders oder über die Ermittlung des aktuellen GPS-Standorts mittels eines Sensors des mobilen Endgeräts erfolgt. Die Identifikation über den Vorgang des automatisierten Scannens weist den Vorteil auf, dass Fehleingaben durch den Benutzer vermieden werden, weiterhin ist der automatisierte Scanvorgang schneller und die Benutzer müssen sich keine Zugangsdaten oder Codes merken, wodurch ebenfalls die Fehleranfälligkeit des Verfahrens weiter reduziert wird.

Als Sensoren des mobilen Endgeräts können beispielsweise die integrierte Kamera bzw. ein Bluetooth-Modul, NFC-Chip bzw. ein GPS-Modul verwendet werden. Durch die Verwendung bereits vorhandener Sensoren wird es ermöglicht, dass jedes handelsübliche mobile Gerät zur Durchführung des Verfahrens bzw. Einbindung in das erfindungsgemäße Verfahren ertüchtigt wird.

Es kann vorgesehen sein, dass an dem Übungsgerät während des Trainings zu erreichende Trainings-Soll-Werte auf dem mobilen Endgerät visuell als grafische Darstellungen vorgegeben werden und anhand der Sensordaten der mindestens einen Sensoreinrichtung in der Darstellung in Echtzeit der erreichte Trainings-Ist-Wert als Echtzeitrückmeldung des Trainingsablaufs wiedergegeben wird.

Bei der grafischen Darstellung kann es sich beispielsweise um eine Straßen-, Balkendarstellung bzw. um eine Tacho-Darstellung handeln. Die Darstellungen erlauben der an dem Übungsgerät trainierenden Person, einen direkten visuellen Abgleich durchzuführen, ob der geforderte Bewegungsablauf während des Trainings erzielt werden konnte bzw. aktuell erzielt wird und weiterhin, ob der geforderte Bewegungsablauf ordnungsgemäß durch den Trainierenden ausgeführt wird. Es ist somit eine unmittelbare Echtzeitkontrolle des Benutzers des Übungsgerätes im Hinblick auf den realisierten Trainingsablauf möglich. Dabei bedarf es nicht der Überwachung durch eine unterwiesene Person oder einem Sportlehrer, vielmehr kann der Nutzer durch die Echtzeitrückmeldung selbst die ordnungsgemäße Durchführung des Trainings unter Einhaltung der zuvor festgelegten Trainingssollwerte sicherstellen.

Insbesondere kann es vorgesehen sein, dass die Ist-Bewegung als dynamische Grafik einer statischen Darstellung des Soll-Bewegungsablaufs überlagert wird.

Es kann somit vorgesehen sein, dass ein Soll-Ist-Vergleich über das erfindungsgemäße Verfahren ermittelt, visualisiert und auch dokumentiert bzw. abgespeichert werden kann. Durch die Dokumentation des Soll-Ist Vergleiches beispielsweise über Speicherung der Trainingsistwerte über einen definierten Zeitraum des Trainings wird es auch nachfolgend des Trainings für den Benutzer ermöglicht das realisierte Training nachzuvollziehen und gegebenenfalls Fehler in Ruhe zu verstehen. Insbesondere ermöglicht die Dokumentation des Soll-Ist-Vergleiches eine spätere Beurteilung des realisierten Trainings durch eine unterwiesene Person, wie einem Sportlehrer um im Hinblick auf zukünftige Trainingseinheiten mögliche Fehler abzustellen und Tipps für ein effektives Training zu geben.

Erfindungsgemäß kann es weiterhin vorgesehen sein, dass anhand der Sensordaten die konzentrische Muskelaktivität bevorzugt mit der entsprechenden Haltephase ermittelt und/oder die exzentrische Muskelaktivität bevorzugt mit der entsprechenden Haltephase und/oder Bewegungsreichweite den zugehörigen Bewegungswendepunkten und auf dem mobilen Endgerät visuell wiedergegeben wird.

Weiterhin kann es vorgesehen sein, dass als Sensordaten die Daten mindestens eines an dem Übungsgerät angeordneten Kraftsensors erfasst werden und auf der Grundlage der vorgenannten Sensordaten die isometrischen und/oder dynamischen Kräfte während des Übungsablaufes in Echtzeit ermittelt werden, über das mobile Endgerät in Echtzeit visualisiert und dokumentiert werden.

Es kann vorgesehen sein, dass die Datenübermittlung zwischen dem mobilen Endgerät und der Auswertungseinheit und/oder der Datenbankeinheit kabellos oder kabelgebunden erfolgt.

Gemäß einem weiteren Aspekt der Erfindung kann ein Überwachungssystem zur Echtzeitüberwachung eines Nutzers eines Trainings an einem Übungsgerät vorgesehen werden, umfassend mindestens ein mobiles Endgerät, mindestens eine an ein Übungsgerät angeordnete Sensoreinrichtung, eine Auswertungseinheit und eine Datenbankeinheit, wobei in der Datenbankeinheit nutzerspezifische Trainingssollwerte, Trainingsrandbedingungen und Übungsgeräte-spezifische Einstellungen abgelegt sind.

In dem erfindungsgemäßen Überwachungssystem kann die mindestens eine Sensoreinrichtung als Entfernungsmesser ausgebildet sein, wobei der Entfernungsmesser derart an dem Übungsgerät angeordnet ist, um den veränderlichen Abstand eines sich während des Trainings bewegenden definierten Objektes des Übungsgeräts gegenüber der unbeweglichen Struktur des Übungsgeräts zu messen. Die mindestens eine Sensoreinrichtung kann als Kraftsensor ausgebildet werden, wobei der Kraftsensor an dem Übungsgerät in dem Lastübertragungspfad zwischen den während des Trainings zu bewegenden Gewichten und/oder Widerstandselementen den Körperauflagerpunkten für den Benutzer an dem Übungsgerät angeordnet ist.

Erfindungsgemäß werden als Körperauflagerpunkte die Elemente an einem Übungsgerät verstanden, über welche der Benutzer während des Trainings die eigene Muskelkraft auf das Übungsgerät überträgt, beispielsweise kann es sich dabei um Handgriffe, Beinauflager oder ähnliches handeln.

In einer bevorzugten Ausführungsform kann es vorgesehen sein, dass die Datenbankeinheit und/oder die Auswertungseinheit als Bestandteil des mobilen Endgerätes der mindestens einen Sensoreinrichtung und/oder einer Zentralrecheneinheit realisiert ist.

Die Datenübertragung zwischen dem mobilen Endgerät der mindestens einen Sensoreinheit und/oder der Zentralrecheneinheit kann dabei drahtlos oder drahtgebunden erfolgen.

Gemäß einem weiteren Aspekt der vorliegenden Erfindung kann ein Trainingssystem umfassend ein Überwachungssystem und ein Übungsgerät zur Durchführung eines Trainings vorgesehen werden.

Ebenfalls kann erfindungsgemäß die Verwendung eines Überwachungssystems oder eines Trainingssystems zur Durchführung des Verfahrens zur Echtzeitüberwachung eines Nutzers an ein Übungsgerät mit einem mobilen Endgerät vorgesehen werden.

Es zeigen:
- Fig. 1: eine schematische Darstellung des Ablaufes und Signalflusses des erfindungsgemäßen Verfahrens zur Echtzeitüberwachung eines Nutzers an einem Übungsgerät, sowie
- Fig. 2: die Kommunikationsflüsse zwischen den Vorrichtungen eines erfindungsgemäßen Überwachungs- bzw. Trainingssystems.

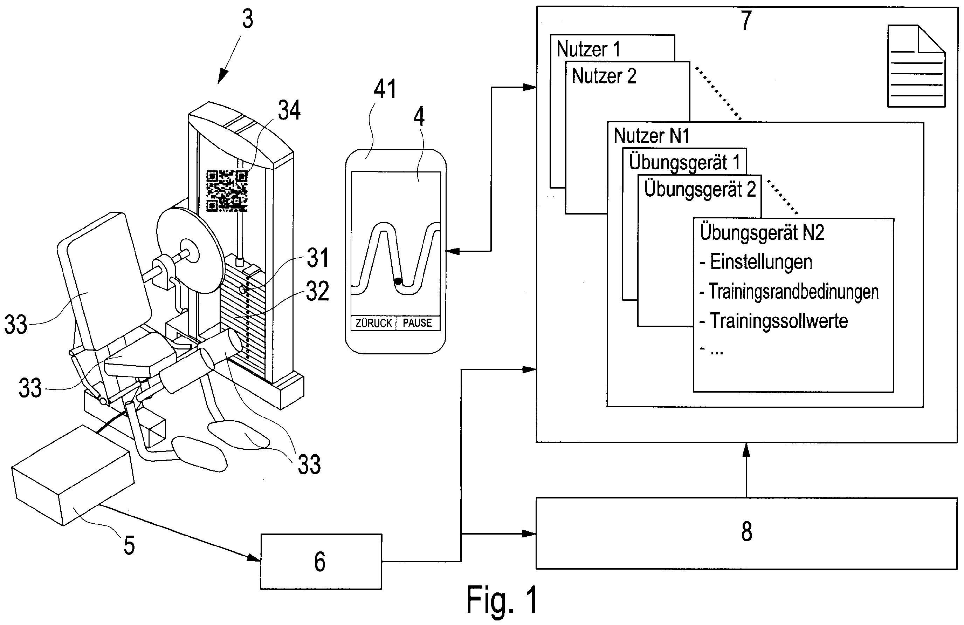

In der Fig. 1 ist ein Übungsgerät 3 dargestellt, an welchem ein Nutzer ein Training absolvieren kann. Die dargestellte beispielhafte Ausführungsform des Übungsgeräts 3 zeigt lediglich als ein mögliches Beispiel eines Übungsgerätes eine kombinierte Adduktions-Abduktions-Maschine. Das vorgenannte Übungsgerät verfügt dabei über eine Mehrzahl von Körperauflagerelementen 33, über welche der trainierende Nutzer die Muskelkraft während des Trainings auf das Übungsgerät 3 übertragen kann. Während des Trainings werden an dem Übungsgerät 3 Widerstandselemente bzw. Gewichtselemente 32 verlagert. Bei der dargestellten Ausführungsform des Übungsgeräts 3 ist eine Einstellung der Gewichtsbelastung für das Training mittels eines Einsteckpins 31 möglich. Weiterhin verfügt das Übungsgerät 3 über einen übungsgerätespezifischen QR-Tag 34, welcher beispielsweise auf dem Übungsgerät 3 aufgeklebt oder an diesem in sonstiger Weise angeordnet ist. An dem Übungsgerät 3 ist weiterhin eine Sensoreinrichtung 5 angeordnet, mittels derer während der Durchführung des Trainings an dem Übungsgerät 3 Sensordaten als Trainings-Ist-Werte 6 erfasst werden, mittels derer beispielsweise der Bewegungsablauf des Trainierenden an dem Übungsgerät 3 oder die aufgebrachte Kraft oder Ähnliches ermittelbar ist. Weiterhin ist in der Fig. 1 ein mobiles Endgerät 4 in Form eines Mobiltelefons, insbesondere Smartphones, dargestellt. Das mobile Endgerät 4 verfügt dabei über einen Sensor 41, wie beispielsweise eine optische Kamera, mittels dessen der QR-Code 32 des Übungsgeräts 3 ausgelesen werden kann. Dabei ist es möglich, dass ein spezifisches Übungsgerät 3 mittels des persönlich mobilen Endgeräts 4 eines trainierenden Nutzers identifiziert wird. In diesem Falle führt beispielsweise der trainierende Nutzer sein persönliches mobiles Endgerät 4 mit und nutzt dieses zur Generierung einer Echtzeitrückmeldung während des Trainings an unterschiedlichen Übungsgeräten. Auf das vorgenannte mobile Endgerät wird dabei zur Einbindung in das erfindungsgemäße Verfahren bzw. in die erfindungsgemäße Vorrichtung eine Software bzw. eine App aufgespielt. Ebenfalls ist es möglich, das mobile Endgerät dauerhaft an dem Übungsgerät 3 anzuordnen und den trainierenden Nutzer beispielsweise durch Eingabe eines Identifizierungscodes über das mobile Endgerät 4 zu identifizieren. In dem vorgenannten Falle verbleibt das mobile Endgerät 4 dauerhaft an einem spezifischen Übungsgerät 3 und wird nicht durch den Nutzer mitgeführt, derart, dass während eines Trainings an unterschiedlichen Übungsgeräten 3 der Nutzer sich an den jeweils an den Übungsgeräten befindlichen mobilen Endgeräten 4 durch Eingabe beispielsweise seines nutzerspezifischen Identifizierungscodes identifizieren muss. Das erfindungsgemäße Verfahren läuft dabei derart ab, dass ein Nutzer vor dem Training an einem Übungsgerät 3 sich mittels seines mitgeführten mobilen Endgeräts 4 an dem spezifischen Übungsgerät 3, an welchem das Training erfolgen soll, identifiziert. Alternativ kann sich der Nutzer ebenfalls über ein an dem Übungsgerät 3, an welchem ein Training erfolgen soll, dauerhaft angeordnetes mobiles Endgerät 4 identifizieren. In einem weiteren Schritt werden während des Trainings mittels mindestens einer an dem Übungsgerät 3 angeordneten Sensoreinrichtung 5 während der Durchführung des Trainings Sensordaten erfasst. Die Sensordaten werden dabei von der Sensoreinrichtung als Trainingsistwerte 6 an eine Datenbankeinheit 7 zum Abgleichen der Sensordaten als Trainingsistwerte 6 mit in einer Datenbankeinheit 7 abgelegten nutzerspezifischen Trainingssollwerten für das Übungsgerät 3 zur Generierung einer Echtzeitrückmeldung des Trainingsablaufes weitergeleitet. Wie dies der Fig. 1 entnehmbar ist, verfügt die Datenbankeinheit zunächst über eine Vielzahl von Nutzerdaten, welche in der Fig. 1 als Nutzer 1, Nutzer 2 bis Nutzer n1 dargestellt sind, wobei es sich bei der Variabel n1 um die Gesamtanzahl der trainierenden Nutzer handelt. In jedem Nutzerdatensatz, beispielhaft für den Nutzer n1 in Fig. 1 dargestellt, sind wiederum eine Vielzahl von Datensätzen im Hinblick auf die unterschiedlichen Übungsgeräte 3 abgelegt. Die unterschiedlichen Übungsgeräte sind dabei in der Fig. 1 zur Unterscheidung als A, B bis N2 bezeichnet, wobei wiederum eine Vielzahl von Datensätzen in der Datenbankeinheit abgelegt ist, welche der Gesamtanzahl der unterschiedlichen Übungsgeräte N2 entspricht. In dem Datensatz der jeweiligen Übungsgeräte sind dann für den spezifischen Nutzer ausgewählte Einstellungen und Trainingsrandbedingungen sowie die Trainings-Soll-Werte abgelegt. Die Echtzeitrückmeldung wird von der Datenbankeinheit 7 an das mobile Endgerät 4 übermittelt, zum Anleiten des Nutzers durch Ausgeben der Echtzeitrückmeldung zur Optimierung des Trainingsablaufes über das mobile Endgerät 4. Nach der Identifikation des Nutzers über das mobile Endgerät 4 kann es ebenfalls erfindungsgemäß vorgesehen sein, dass vor Beginn des eigentlichen Trainings auf der Datenbankeinheit 7 abgelegte Trainingsrandbedingungen vorzunehmende Einstellungen des jeweiligen spezifischen Übungsgerätes 3 vor Trainingsbeginn über das mobile Endgerät wiedergegeben werden. Der Signalfluss ist dabei über den Doppelpfeil zwischen dem mobilen Endgerät 4 und der Datenbankeinheit 7 dargestellt. Dabei überwacht eine Auswertungseinheit 8 die Vornahme der vorzunehmenden Einstellungen durch Abgleichen der Sensordaten mit den auf der Datenbankeinheit abgelegten Trainingsrandbedingungen und vorzunehmenden Einstellungen.

Nach Abschluss der Vornahme der Einstellungen kann das Training für den Benutzer über das mobile Endgerät 4 freigegeben werden.

Wie dies ebenfalls der Fig. 1 entnehmbar ist, können die Trainingsistwerte 6 vor dem Abgleichen der Sensordaten durch die Datenbankeinheit 7 mittels einer Auswertungseinheit interpretiert gefiltert und sortiert werden und in einem nutzerspezifischen Datensatz der Datenbankeinheit 7 abgelegt und darauffolgend der Datensatz mit den nutzerspezifischen Trainingssollwerten abgeglichen werden und zur Generierung der Echtzeitrückmeldung über das mobile Endgerät 4 genutzt werden.

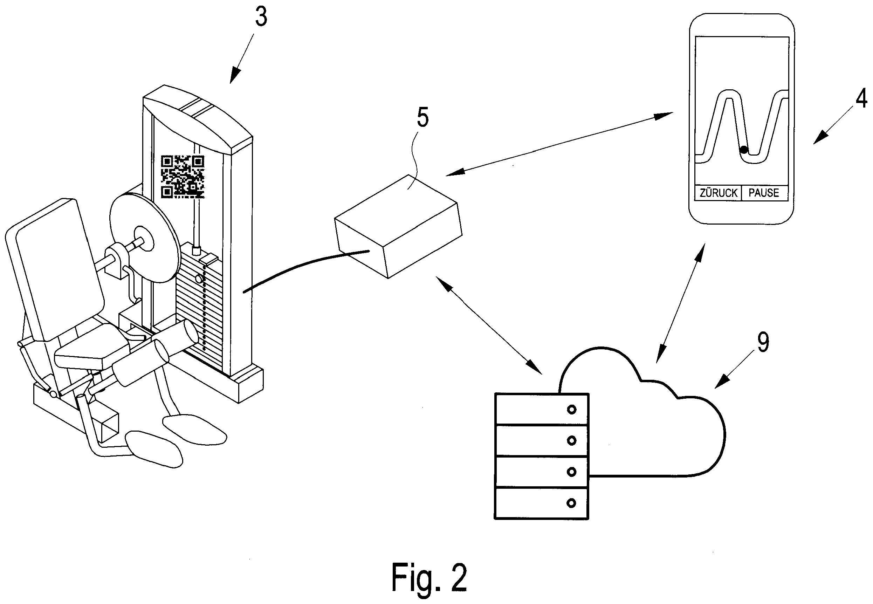

Die Fig. 2 zeigt die Kommunikationswege zwischen der an dem Übungsgerät 3 angeordneten Sensoreinrichtung 5 sowie dem mobilen Endgerät 4 und einer optional vorgesehenen Zentralrecheneinheit 9. Wie dies der Fig. 2 entnehmbar ist, können die vorgenannte Sensoreinrichtung 5, das mobile Endgerät 4 sowie die optionale Einrichtung 9 jeweils miteinander kommunizieren. Als Kommunikationsform kann eine drahtgebundene und/oder drahtlose Kommunikation mit üblichen Übertragungsstandards vorgesehen werden.

## Patentansprüche

1. Verfahren zur Echtzeitüberwachung eines Nutzers an einem Übungsgerät (3) mit einem mobilen Endgerät (4), umfassend die Verfahrensschritte:
a) Identifizieren des Übungsgerätes (3), an welchem ein Training erfolgen soll, mittels des mobilen Endgeräts (4) des Nutzers oder Identifizieren des Nutzers über ein an dem Übungsgerät (3), an welchem ein Training erfolgen soll, angeordnetes mobiles Endgerät (4),
b) Erfassen von Sensordaten mit mindestens einer an dem Übungsgerät (3) angeordneten Sensoreinrichtung (5) während der Durchführung des Trainings,
c) Abgleichen der Sensordaten als Trainings-Ist-Werte (6) mit in einer Datenbankeinheit (7) abgelegten nutzerspezifischen Trainings-Soll-Werten für das Übungsgerät (3) zur Generierung einer Echtzeitrückmeldung des Trainingsablaufes, und
d) Anleiten des Nutzers durch Ausgeben der Echtzeitrückmeldung zur Optimierung des Trainingsablaufes über das mobile Endgerät (4).

2. Verfahren nach Anspruch 1, **dadurch gekennzeichnet, dass** das Verfahren nach dem Verfahrensschritt a) und vor dem Verfahrensschritt b) die zusätzlichen Verfahrensschritte umfasst:
- Wiedergeben von auf der Datenbankeinheit (7) abgelegten Trainingsrandbedingungen und vorzunehmender Einstellungen am Übungsgerät (3) vor Trainingsbeginn über das mobile Endgerät (4),
- Überwachen der Vornahme der vorzunehmenden Einstellungen durch Abgleichen der Sensordaten mit den auf der Datenbankeinheit (7) abgelegten Trainingsrandbedingungen und vorzunehmender Einstellungen mittels einer Auswertungseinheit (8), und
- Freigeben des Trainings nach Abschluss der Vornahme der Einstellungen über das mobile Endgerät (4).

3. Verfahren nach Anspruch 1 oder 2, **dadurch gekennzeichnet, dass** im Verfahrensschritt c) vor dem Abgleichen der Sensordaten durch die Datenbankeinheit (7) die Sensordaten mittels einer Auswertungseinheit (8) interpretiert, gefiltert und sortiert werden, und in einem nutzerspezifischen Datensatz der Datenbankeinheit (7) abgelegt und darauffolgend der Datensatz mit den nutzerspezifischen Trainings-Soll-Werten zur Generierung der Echtzeitrückmeldung abgeglichen wird.

4. Verfahren nach einem der vorangehenden Ansprüche, **dadurch gekennzeichnet, dass** als Sensordaten die Position und/oder die zurückgelegten Wege eines definierten Objektes (31) des Übungsgerätes (3) und/oder die Geschwindigkeit, Winkellage oder Beschleunigung eines definierten Objektes (31) des Übungsgerätes (3) über die Zeitdauer des Trainingsablaufes mittels der mindestens einen Sensoreinrichtung (5) erfasst werden.

5. Verfahren nach einem der vorangehenden Ansprüche, **dadurch gekennzeichnet, dass** die Ausgabe der Echtzeitrückmeldung des Trainingsablaufes, der Trainingsrandbedingungen und/oder vorzunehmender Einstellungen optisch, haptisch und/oder akustisch über das mobile Endgerät (4) erfolgt.

6. Verfahren nach einem der vorangehenden Ansprüche, **dadurch gekennzeichnet, dass** es sich bei der mindestens einen Sensoreinrichtung (5) um einen Kraftsensor, Entfernungssensor, Geschwindigkeitssensor, Rotationssensor, Winkelsensor und/oder Beschleunigungssensor handelt.

7. Verfahren nach einem der vorangehenden Ansprüche, **dadurch gekennzeichnet, dass** die Echtzeitüberwachung bei der Durchführung eines Trainings mit mindestens einem kardiovaskulären Trainingsanteil erfolgt, wobei im Verfahrensschritt b) als zusätzliche Sensordaten mindestens ein Leistungsparameter am Übungsgerät (3) erfasst wird, wobei der mindestens eine Leistungsparameter ausgewählt ist aus der Gruppe von:
- Umdrehungszahl,
- Watt,
- Steigung,
- Geschwindigkeit,
- Beschleunigung,
- Gang/Stufe,
- Puls,
- berechneter Kalorienverbrauch (kcal),
- Trainingsdauer,
- aufgenommene Sauerstoffmenge (VO2) in Liter pro Minute,
- aufgenommene maximale Sauerstoffmenge (VO2max),
- Seitigkeit links/rechts, und/oder
- Wiederholungen,
und wobei der mindestens eine Leistungsparameter als ergänzende Information im Verfahrensschritt d) bevorzugt ohne vorherige Durchführung des Verfahrensschrittes c) dem Nutzer über das mobile Endgerät (4) zusätzlich als Echtzeitrückmeldung ausgegeben wird.

8. Verfahren nach einem der vorangehenden Ansprüche, **dadurch gekennzeichnet, dass** das Identifizieren des Übungsgerätes (3) oder des Nutzers über die manuelle Eingabe eines Identifikationscodes an dem mobilen Endgerät (4) oder über das Scannen eines QR-Codes (32), NFC-Tags oder eines Transponders oder über die Ermittlung des aktuellen GPS-Standortes mittels eines Sensors (41) des mobilen Endgerätes (4) erfolgt.

9. Verfahren nach einem der vorangehenden Ansprüche, **dadurch gekennzeichnet, dass** der an dem Übungsgerät während des Trainings zu erreichende Trainings-Soll-Wert auf dem mobilen Endgerät (4) visuell als grafische Darstellung vorgegeben wird und anhand der Sensordaten der mindestens einen Sensoreinrichtung (5) in der Darstellung in Echtzeit der erzielte Trainings-Ist-Wert als Echtzeitrückmeldung des Trainingsablaufs wiedergegeben wird.

10. Überwachungssystem zur Echtzeitüberwachung eines Nutzers während eines Trainings an einem Übungsgerät (3), umfassend:
- mindestens ein mobiles Endgerät (4),
- mindestens eine an einem Übungsgerät (3) angeordnete Sensoreinrichtung (5),
- eine Datenbankeinheit (7), wobei in der Datenbankeinheit (7) nutzerspezifische Trainings-Soll-Werte, Trainingsrandbedingungen und übungsgerätespezifische Einstellungen abgelegt sind, und
- bevorzugt eine Auswertungseinheit (8).

11. Überwachungssystem nach Anspruch 10, **dadurch gekennzeichnet, dass** die mindestens eine Sensoreinrichtung (5) als Entfernungsmesser ausgebildet ist, wobei der Entfernungsmesser derart an dem Übungsgerät (3) angeordnet ist, um den veränderlichen Abstand eines sich während des Trainings bewegenden definierten Objekts (31) des Übungsgerätes (3) gegenüber der unbeweglichen Struktur des Übungsgerätes (3) zu messen.

12. Überwachungssystem nach Anspruch 10 oder 11, **dadurch gekennzeichnet, dass** die mindestens eine Sensoreinrichtung (5) als Kraftsensor ausgebildet ist, wobei der Kraftsensor an dem Übungsgerät (3) in dem Lastübertragungspfad zwischen den während des Trainings zu bewegenden Gewichten (32) und/oder Widerstandselementen und den Körperauflagerelementen (33) für den Benutzer an dem Übungsgerät (3) angeordnet ist.

13. Überwachungssystem nach einem der Ansprüche 10 bis 12, **dadurch gekennzeichnet, dass** die Datenbankeinheit (7) und/oder die Auswertungseinheit (8) als Bestandteil des mobilen Endgerätes (4) der mindestens einen Sensoreinrichtung (5) und/oder einer Zentralrecheneinheit realisiert ist.

14. Trainingssystem umfassend ein Überwachungssystem nach einem der Ansprüche 10 bis 13 und ein Übungsgerät (3) zur Durchführung eines Trainings.

15. Verwendung eines Überwachungssystems gemäß einem der Ansprüche 10 bis 13 oder eines Trainingssystems gemäß Anspruch 14 zur Durchführung des Verfahrens gemäß einem der Ansprüche 1 bis 9.

## Geänderte Patentansprüche

### Geänderte Patentansprüche gemäss Regel 137(2) EPÜ.

1. Verfahren zur Echtzeitüberwachung eines Nutzers an einem Übungsgerät (3) mit einem mobilen Endgerät (4), umfassend die Verfahrensschritte:
a) Identifizieren des Übungsgerätes (3), an welchem ein Training erfolgen soll, mittels des mobilen Endgeräts (4) des Nutzers oder Identifizieren des Nutzers über ein an dem Übungsgerät (3), an welchem ein Training erfolgen soll, angeordnetes mobiles Endgerät (4),
b) Erfassen von Sensordaten mit mindestens einer an dem Übungsgerät (3) angeordneten Sensoreinrichtung (5) während der Durchführung des Trainings,
c) Abgleichen der Sensordaten als Trainings-Ist-Werte (6) mit in einer Datenbankeinheit (7) abgelegten nutzerspezifischen Trainings-Soll-Werten für das Übungsgerät (3) zur Generierung einer Echtzeitrückmeldung des Trainingsablaufes, und
d) Anleiten des Nutzers durch Ausgeben der Echtzeitrückmeldung zur Optimierung des Trainingsablaufes über das mobile Endgerät (4), wobei der an dem Übungsgerät während des Trainings zu erreichende Trainings-Soll-Wert auf dem mobilen Endgerät (4) visuell als grafische Darstellung, beispielsweise in Form einer Straßendarstellung, vorgegeben wird und anhand der Sensordaten der mindestens einen Sensoreinrichtung (5) in der Darstellung in Echtzeit der erzielte Trainings-Ist-Wert als Echtzeitrückmeldung des Trainingsablaufs wiedergegeben wird.

2. Verfahren nach Anspruch 1, **dadurch gekennzeichnet, dass** das Verfahren nach dem Verfahrensschritt a) und vor dem Verfahrensschritt b) die zusätzlichen Verfahrensschritte umfasst:
- Wiedergeben von auf der Datenbankeinheit (7) abgelegten Trainingsrandbedingungen und vorzunehmender Einstellungen am Übungsgerät (3) vor Trainingsbeginn über das mobile Endgerät (4),
- Überwachen der Vornahme der vorzunehmenden Einstellungen durch Abgleichen der Sensordaten mit den auf der Datenbankeinheit (7) abgelegten Trainingsrandbedingungen und vorzunehmender Einstellungen mittels einer Auswertungseinheit (8), und
- Freigeben des Trainings nach Abschluss der Vornahme der Einstellungen über das mobile Endgerät (4).

3. Verfahren nach Anspruch 1 oder 2, **dadurch gekennzeichnet, dass** im Verfahrensschritt c) vor dem Abgleichen der Sensordaten durch die Datenbankeinheit (7) die Sensordaten mittels einer Auswertungseinheit (8) interpretiert, gefiltert und sortiert werden, und in einem nutzerspezifischen Datensatz der Datenbankeinheit (7) abgelegt und darauffolgend der Datensatz mit den nutzerspezifischen Trainings-Soll-Werten zur Generierung der Echtzeitrückmeldung abgeglichen wird.

4. Verfahren nach einem der vorangehenden Ansprüche, **dadurch gekennzeichnet, dass** als Sensordaten die Position und/oder die zurückgelegten Wege eines definierten Objektes (31) des Übungsgerätes (3) und/oder die Geschwindigkeit, Winkellage oder Beschleunigung eines definierten Objektes (31) des Übungsgerätes (3) über die Zeitdauer des Trainingsablaufes mittels der mindestens einen Sensoreinrichtung (5) erfasst werden.

5. Verfahren nach einem der vorangehenden Ansprüche, **dadurch gekennzeichnet, dass** die Ausgabe der Echtzeitrückmeldung des Trainingsablaufes, der Trainingsrandbedingungen und/oder vorzunehmender Einstellungen optisch, haptisch und/oder akustisch über das mobile Endgerät (4) erfolgt.

6. Verfahren nach einem der vorangehenden Ansprüche, **dadurch gekennzeichnet, dass** es sich bei der mindestens einen Sensoreinrichtung (5) um einen Kraftsensor, Entfernungssensor, Geschwindigkeitssensor, Rotationssensor, Winkelsensor und/oder Beschleunigungssensor handelt.

7. Verfahren nach einem der vorangehenden Ansprüche, **dadurch gekennzeichnet, dass** die Echtzeitüberwachung bei der Durchführung eines Trainings mit mindestens einem kardiovaskulären Trainingsanteil erfolgt, wobei im Verfahrensschritt b) als zusätzliche Sensordaten mindestens ein Leistungsparameter am Übungsgerät (3) erfasst wird, wobei der mindestens eine Leistungsparameter ausgewählt ist aus der Gruppe von:
- Umdrehungszahl,
- Watt,
- Steigung,
- Geschwindigkeit,
- Beschleunigung,
- Gang/Stufe,
- Puls,
- berechneter Kalorienverbrauch (kcal),
- Trainingsdauer,
- aufgenommene Sauerstoffmenge (VO2) in Liter pro Minute,
- aufgenommene maximale Sauerstoffmenge (VO2max),
- Seitigkeit links/rechts, und/oder
- Wiederholungen,
und wobei der mindestens eine Leistungsparameter als ergänzende Information im Verfahrensschritt d) bevorzugt ohne vorherige Durchführung des Verfahrensschrittes c) dem Nutzer über das mobile Endgerät (4) zusätzlich als Echtzeitrückmeldung ausgegeben wird.

8. Verfahren nach einem der vorangehenden Ansprüche, **dadurch gekennzeichnet, dass** das Identifizieren des Übungsgerätes (3) oder des Nutzers über die manuelle Eingabe eines Identifikationscodes an dem mobilen Endgerät (4) oder über das Scannen eines QR-Codes (32), NFC-Tags oder eines Transponders oder über die Ermittlung des aktuellen GPS-Standortes mittels eines Sensors (41) des mobilen Endgerätes (4) erfolgt.

9. Überwachungssystem zur Echtzeitüberwachung eines Nutzers während eines Trainings an einem Übungsgerät (3), umfassend:
- mindestens ein mobiles Endgerät (4),
- mindestens eine an einem Übungsgerät (3) angeordnete Sensoreinrichtung (5),
- eine Datenbankeinheit (7), wobei in der Datenbankeinheit (7) nutzerspezifische Trainings-Soll-Werte, Trainingsrandbedingungen und übungsgerätespezifische Einstellungen abgelegt sind, und
- bevorzugt eine Auswertungseinheit (8), **dadurch gekennzeichnet, dass** auf dem mobilen Endgerät (4) der an dem Übungsgerät während des Trainings zu erreichende Trainings-Soll-Wert visuell als grafische Darstellung, beispielsweise in Form einer Straßendarstellung, vorgegeben wird und anhand der Sensordaten der mindestens einen Sensoreinrichtung (5) in der Darstellung in Echtzeit der erzielte Trainings-Ist-Wert als Echtzeitrückmeldung des Trainingsablaufs wiedergegeben wird.

10. Überwachungssystem nach Anspruch 9, **dadurch gekennzeichnet, dass** die mindestens eine Sensoreinrichtung (5) als Entfernungsmesser ausgebildet ist, wobei der Entfernungsmesser derart an dem Übungsgerät (3) angeordnet ist, um den veränderlichen Abstand eines sich während des Trainings bewegenden definierten Objekts (31) des Übungsgerätes (3) gegenüber der unbeweglichen Struktur des Übungsgerätes (3) zu messen.

11. Überwachungssystem nach Anspruch 9 oder 10, **dadurch gekennzeichnet, dass** die mindestens eine Sensoreinrichtung (5) als Kraftsensor ausgebildet ist, wobei der Kraftsensor an dem Übungsgerät (3) in dem Lastübertragungspfad zwischen den während des Trainings zu bewegenden Gewichten (32) und/oder Widerstandselementen und den Körperauflagerelementen (33) für den Benutzer an dem Übungsgerät (3) angeordnet ist.

12. Überwachungssystem nach einem der Ansprüche 9 bis 11, **dadurch gekennzeichnet, dass** die Datenbankeinheit (7) und/oder die Auswertungseinheit (8) als Bestandteil des mobilen Endgerätes (4) der mindestens einen Sensoreinrichtung (5) und/oder einer Zentralrecheneinheit realisiert ist.

13. Trainingssystem umfassend ein Überwachungssystem nach einem der Ansprüche 9 bis 12 und ein Übungsgerät (3) zur Durchführung eines Trainings.

14. Verwendung eines Überwachungssystems gemäß einem der Ansprüche 9 bis 12 oder eines Trainingssystems gemäß Anspruch 13 zur Durchführung des Verfahrens gemäß einem der Ansprüche 1 bis 8.
